# EUROPEAN PATENT SPECIFICATION

(11) **EP 4 067 341 B1**
(45) Date of publication and mention of the grant of the patent: **03.07.2024**
(21) Application number: 19954373.7
(22) Date of filing: 29.11.2019
(51) Int. Cl.: C07C 319/14, C07C 323/58

(54) **METHOD FOR PREPARING CARBOCISTEINE**
VERFAHREN ZUR HERSTELLUNG VON CARBOCISTEIN
PROCÉDÉ DE PRÉPARATION DE CARBOCISTÉINE

(43) Date of publication of application: 05.10.2022
(73) Proprietor: Wuhan Grand Hoyo Co., Ltd., Wuhan, Hubei 430074 (CN)
(72) Inventor: HUANG, Lei, Wuhan, Hubei 430074 (CN); LI, Jing, Wuhan, Hubei 430074 (CN); FAN, Pei, Wuhan, Hubei 430074 (CN); ZHU, Chengjun, Wuhan, Hubei 430074 (CN); YANG, Lei, Wuhan, Hubei 430074 (CN); SHU, Min, Wuhan, Hubei 430074 (CN); TANG, Peng, Wuhan, Hubei 430074 (CN); HE, Jiajun, Wuhan, Hubei 430074 (CN); GUO, Liping, Wuhan, Hubei 430074 (CN); MEI, Xuechen, Wuhan, Hubei 430074 (CN); HUANG, Jiaqi, Wuhan, Hubei 430074 (CN)
(74) Representative: Greaves Brewster LLP
(86) International application number: PCT/CN2019/122003
(87) International publication number: WO 2021/102918

(56) References cited:
- CN-A- 103 102 294
- CN-A- 103 588 662
- CN-A- 105 418 471
- CN-A- 106 083 673
- CN-A- 106 565 565
- CN-A- 106 565 565
- CN-A- 106 748 926
- CN-A- 109 053 508
- CN-A- 109 053 508
- CN-A- 110 452 142
- CN-A- 110 452 142
- CN-C- 1 159 474
- JP-A- S62 135 455
- Yang Xiao-yun, Yang Zhi: "The improvement of Carbocisteine s Synthesis Technology", Shangdong Pharmaceutical Industry, vol. 23, no. 3, 30 March 2004 (2004-03-30) , pages 43-44, XP055814577, ISSN: 2095-5375
- Huang Keming: "An Improvement on the Purification Technology of Carboxymethyl Cysteine", Journal of Guilin Medical University, vol. 9, no. 1, 15 February 1996 (1996-02-15), pages 55-56, XP055814585, ISSN: 1008-2409
- Gu Yongqing: "Improvement of the synthetic method of S-carboxymethylcysteine", Chinese Pharmaceutical Journal, vol. 28, no. 12, 8 December 1993 (1993-12-08), pages 744-745, XP055814589, CN ISSN: 1001-2494

## Description

### TECHNICAL FIELD

The present disclosure relates to a method for preparing carbocisteine.

### BACKGROUND

Carbocisteine (Carbocistein) has a chemical name of S-carboxymethyl-*L*-cysteine, a molecular formula: C₅H₉NO₄S, a molecular weight: 179.19, and a structural formula: 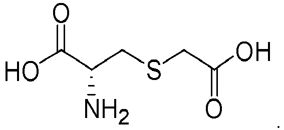 This product is white crystalline powder; and odorless. This product is slightly soluble in hot water, very slightly soluble in water, insoluble in ethanol or acetone; easily soluble in an acid or base solution.

Carbocisteine is a viscous phlegm dissolving drug, which can reduce the secretion of mucus in the gas transport tube, reduce the viscosity of sputum and make it easier to cough up. Carbocisteine is clinically used for thick sputum, expectoration difficulty and pulmonary ventilation insufficiency caused by chronic bronchitis and bronchial asthma, etc.

The synthesis of carbocisteine in a traditional batch ceramic reactor is the mainstream method for producing carbocisteine at present, and the method has the advantages of low cost and high productivity. However, it is difficult to accurately control the reaction temperature, reaction time, reaction pH and other conditions in the traditional reactor, and the product quality (for example, a large amount of impurities generated) and product yield fluctuate greatly, resulting in a certain probability of unqualified products. Therefore, there is an urgent need to develop a method for preparing carbocisteine. The product obtained by this method has high quality, small fluctuation of product yield, and is easy to operate and control.

CN106565565A discloses a method for preparing carbocisteine. In the method for preparing, L-cysteine hydrochloride is used as a raw material to react with chloroacetic acid, and the content of impurities in the product is reduced by adding carbonate. The reaction equation is shown as follows:

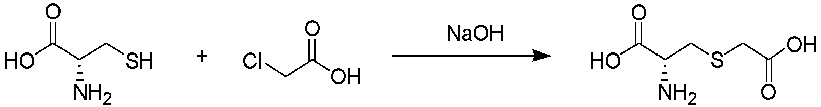

In this patent, only the content of an impurity, cystine, can be reduced, and additional antioxidants and carbonates are required to be added.

### CONTENT OF THE PRESENT INVENTION

The present disclosure provides a method for preparing carbocisteine different from the prior art. The method for preparing of the present disclosure produces a small amount of impurities, and the prepared carbocisteine can meet the CEP registration standards through conventional post-treatment.

In the prior art, there is little research on how to reduce the impurity content in carbocisteine, except that the above CN106565565A mentions that it can reduce the content of cystine, and there is almost no report on how to reduce the impurity content. In the process of research, the inventor of the present disclosure found that the impurity content in carbocisteine can be well controlled by using the reaction of a tiny unit and controlling the size of a reaction channel.

The present disclosure solves the above technical problem through the following technical solutions.

The present disclosure provides a method for preparing carbocisteine, comprising the following steps: in a continuous-flow reactor, carrying out a condensation reaction as shown below between *L*-cysteine as shown in formula I and a compound as shown in formula II in the presence of a base and in a solvent to obtain the carbocisteine as shown in formula III;
wherein, the size of a reaction channel of the continuous-flow reactor is 0.01-200 mm; a molar ratio of the compound as shown in formula II to the *L*-cysteine as shown in formula I is 0.95: 1 -1.10: 1; the temperature of the condensation reaction is 35 to 75 °C; a residence time of the condensation reaction is 2.08-16.67 min; 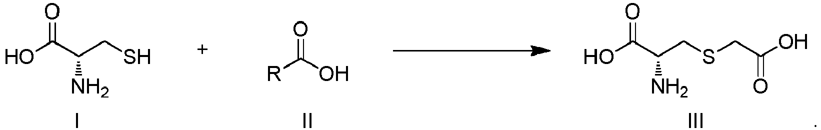 R is methyl substituted by halogen.

In the present disclosure, in formula II, when R is the methyl substituted by halogen, the halogen may be F, Cl, Br or I, and may also be Cl.

In the present disclosure, in formula II, when R is the methyl substituted by halogen, the number of the substitutions may be 1.

The continuous-flow reactor is a reactor with high throughput and easy to scale up production scale.

In the present disclosure, the size of the reaction channel of the continuous-flow reactor refers to the diameter of the reaction channel.

In the present disclosure, the continuous-flow reactor may be a micro-reactor or a tubular reactor, and may also be a micro channel reactor. The micro-reactor may be a conventional type of micro-reactor suitable for such liquid-liquid homogeneous reactions in the art. In the present disclosure, Corning micro-reactor LF, Wenhao WH-IND MT280 or Wuhan Shengshi Fine Chemicals Floway F1 may be selected, or Wuhan Shengshi Fine Chemicals Floway F1 may also be selected.

The micro-reactor, that is, micro-channel reactor, is a micro-reactor with a characteristic size between 10 and 3000 microns manufactured by precision machining technology. The micro-reactor can contain millions or tens of millions of micro-channels, which can achieve a high yield. The internal microstructure of the micro-reactor makes the micro-reactor equipment have a huge specific surface area, which can reach hundreds or even thousands of times of the specific surface area of a stirred tank.

The reaction conditions and reaction parameters of the condensation reaction may be the conventional reaction conditions and reaction parameters for such reactions in the art. The following reaction conditions and parameters are particularly preferred in the present disclosure:

a reagent used in the condensation reaction may not include one or more of Na₂CO₃, NaHCO₃ and K₂CO₃, and further may not include one or more of Na₂SO₃ and/or Na₂S₂O₅. In the present disclosure, the excluded reagents are not limited to Na₂CO₃, NaHCO₃, K₂CO₃, KHCO₃, Na₂SO₃ or Na₂S₂O₅. As long as in the system of the present disclosure, carbonates which can achieve similar effects as Na₂CO₃, NaHCO₃, K₂CO₃ or KHCO₃, and antioxidants which have similar effects as Na₂SO₃ or Na₂S₂O₅ belong to the above-mentioned reagents.

In the condensation reaction, the base can be a base conventionally used in such reactions in the art, preferably an alkali metal base and/or an ammonia solution, more preferably the alkali metal base. The alkali metal base may be sodium hydroxide. The ammonia solution may be ammonia water, such as 20 % ammonia water.

In the condensation reaction, the solvent may be a solvent conventionally used in such reactions in the art, and may also be water, such as pure water.

In the condensation reaction, the base is preferably introduced into the continuous-flow reactor in the form of a solution to carry out the condensation reaction.

Wherein, when the base is introduced into the continuous-flow reactor in the form of the solution, a flow rate of the solution may be 1-8 mL/min (for example, 2 mL/min, 4 mL/min, 5 mL/min).

Wherein, when the base is introduced into the continuous-flow reactor in the form of the solution, a molar concentration of the base may be 6-9 mol/L, for example, 8 mol/L.

In the condensation reaction, the *L*-cysteine as shown in formula I refers to *L*-cysteine precursor compound as shown in formula I which can be converted into *L*-cysteine as shown in formula I under the reaction system of the present disclosure (such as a salt and hydrate thereof), preferably a salt of *L*-cysteine and/or *L*-cysteine hydrochloride monohydrate, and may also be *L*-cysteine hydrochloride monohydrate.

In the condensation reaction, the *L*-cysteine as shown in the formula I is preferably introduced into the continuous-flow reactor in the form of a solution to carry out the condensation reaction.

Wherein, when the *L*-cysteine as shown in the formula I is introduced into the continuous-flow reactor in the form of the solution, a flow rate of the solution may be 2-16 mL/min (for example 4 mL /min, 8 mL/min), and may also be 10-16 mL/min.

Wherein, when the *L*-cysteine as shown in formula I is introduced into the continuous-flow reactor in the form of the solution, a molar concentration of the *L*-cysteine as shown in formula I may be 1-3 mol/L, for example, 1.25 mol/L.

In the condensation reaction, the molar ratio of the compound as shown in formula II to the *L*-cysteine as shown in formula I may be 0.95: 1-1.05: 1, preferably 1.00: 1-1.05: 1, such as 1.02: 1.

In the condensation reaction, the compound as shown in formula II may be chloroacetic acid

In the condensation reaction, the compound as shown in formula II is preferably introduced into the continuous-flow reactor in the form of a solution to carry out the condensation reaction.

When the compound as shown in formula II is introduced into the continuous-flow reactor in the form of the solution, the flow rate of the solution may be 2-16 mL/min (for example, 4 mL/min, 8 mL/min), and may also be 10-16 mL/min.

When the compound as shown in formula II is introduced into the continuous-flow reactor in the form of the solution, a molar concentration of the compound as shown in formula II may be 1.1875-1.3125 mol/L, for example 1.25 mol/L, 1.275 mol/L.

In the condensation reaction, a molar ratio of the base to the *L*-cysteine as shown in formula I may be 3:1-4:1, for example, 3.2:1.

The temperature of the condensation reaction may be 35±1 to 65±1 °C, preferably 35±1 to 55±1 °C.

The residence time of the condensation reaction is a conventional term in the art, which generally refers to the residence time of the reaction material in the micro-channel reactor from entering the micro-channel reactor. In the present disclosure, the residence time refers to the residence time of the base, the *L*-cysteine as shown in formula I, the compound as shown in formula II and the solvent in the continuous-flow reactor. The monitoring method for the progress of the condensation reaction can be carried out by the conventional monitoring method in the art (such as starch chromogenic method). Generally, the color of the reaction solution indicating that the raw material, that is, *L*-cysteine as shown in formula I, has been completely converted is taken as an end point of the reaction.

The residence time of the condensation reaction may be 2.08 to 3.33 min.

In an embodiment of the present disclosure, the method for preparing comprises the following steps: mixing the *L*-cysteine as shown in formula I, the compound as shown in formula II and the solvent to obtain a solution I-II, mixing the base and the solvent to obtain a solution V, and pumping the solution I-II and the solution V into the continuous-flow reactor respectively; wherein the continuous-flow reactor is the micro-reactor.

In an embodiment of the present disclosure, the method for preparing comprises the following steps: mixing the *L*-cysteine as shown in formula I, the compound as shown in formula II and the solvent to obtain the solution I-II, mixing the base and the solvent to obtain the solution V, pumping the solution I-II and the solution V into the continuous-flow reactor respectively, setting a reaction temperature, controlling the flow rates of the solution I-II and the solution V, controlling the flow rate ratio of the solution I-II to the solution V, and completing the reaction within the reaction residence time; wherein, the continuous-flow reactor is the micro-reactor; the molar ratios of the *L*-cysteine as shown in formula I to the compound as shown in formula II, the reaction temperature, the flow rates of the solution I-II and the solution V, the flow rate ratio of the solution I-II to the solution V, and the residence time are all as described above.

In the present disclosure, the condensation reaction preferably further comprises a post-treatment process, and the post-treatment process may use the conventional post-treatment operations and conditions of such reactions in the art. The post-treatment of the present disclosure comprises the following steps: diluting, adjusting the pH value to 2.5-3.0, cooling, recrystallizing, filtering and drying.

In the step of diluting, the operations and methods for diluting may be conventional operations and methods in the art. The solvent used for diluting may be water. A volume mass ratio of the solvent for diluting to the *L*-cysteine as shown in formula I is preferably 7-10 mL/g.

In the step of adjusting the pH value to 2.5-3.0, the operations and methods for adjusting the pH value to 2.5-3.0 may be conventional operations and methods in the art. The method for adjusting the pH value to 2.5-3.0 may be adjusted by a conventional method in the art, preferably by a regulator. The regulator may be a conventional regulator for such operations, preferably an inorganic acid. The inorganic acid is preferably hydrochloric acid.

Before the step of adjusting the pH value to 2.5-3.0, a reaction solution after diluting is preferably heated to 50 to 70 °C, for example, 60 °C.

In the step of cooling, a temperature for cooling may be 20 to 35 °C. After the step of cooling, filtering and water washing may also be comprised to obtain a crude product of carbocisteine.

In the step of recrystallizing, the operation and conditions for recrystallizing may be conventional operations and conditions in the art. The solvent used for recrystallizing may be a conventional solvent for recrystallizing in the art, preferably water. A volume mass ratio of the solvent used for recrystallizing to the crude product of the carbocisteine may be 6-7 mL/g. A temperature for recrystallizing may be 50 to 55 °C. The recrystallizing may further comprise a step of decolorizing. Preferably, decolorizing with an activated carbon in the solvent. Preferably, after the step of decolorizing, a pH value of the recrystallized solution is adjusted to 2-2.5, for example, 2.3. The amount of the activated carbon may be the conventional amount for such operations in the art, preferably 1.5 wt %, and the amount is the weight percentage of the mass of the activated carbon to the mass of the crude product of carbocisteine.

Wherein, the conditions and operations for filtering may be conventional conditions and operations in the art, preferably filtering under reduced pressure.

Wherein, the conditions and operations for drying may be conventional conditions and operations in the art.

The present disclosure also provides a carbocisteine prepared by the above method for preparing.

The present disclosure also provides a carbocisteine composition, wherein, the content of cystine is 0.01-0.1 %, the content of chloroacetic acid is 0-0.04 %, the content of carbocisteine lactam is 0-0.02 %, the content of N-carboxymethyl-*S*-carboxymethyl cysteine is 0.03-0.15 % and the content of impurity cysteine is 0.03-0.87 %, and the remaining content is carbocisteine; the content is a mass percentage of the mass of each component to a total mass of carbocisteine and each component.

The carbocisteine composition is optionally any one of the following:
the content of cystine is 0.03 %, the content of chloroacetic acid is 0, the content of carbocisteine lactam is 0, the content of *N*-carboxymethyl-*S*-carboxymethyl cysteine is 0.03 % and the content of impurity cysteine is 0.87 %, and the remaining content is carbocisteine; the content is the mass percentage of the mass of each component to the total mass of carbocisteine and each component;
the content of cystine is 0.05 %, the content of chloroacetic acid is 0, the content of carbocisteine lactam is 0, the content of *N*-carboxymethyl-*S*-carboxymethyl cysteine is 0.01 % and the content of impurity cysteine is 0.04 %, and the remaining content is carbocisteine; the content is the mass percentage of the mass of each component to the total mass of carbocisteine and each component;
the content of cystine is 0.06 %, the content of chloroacetic acid is 0, the content of carbocisteine lactam is 0, the content of N-carboxymethyl-S-carboxymethyl cysteine is 0.12 % and the content of impurity cysteine is 0.01 %, and the remaining content is carbocisteine; the content is the mass percentage of the mass of each component to the total mass of carbocisteine and each component;
the content of cystine is 0.05 %, the content of chloroacetic acid is 0, the content of carbocisteine lactam is 0, the content of *N*-carboxymethyl-*S*-carboxymethyl cysteine is 0.12 % and the content of impurity cysteine is 0, and the remaining content is carbocisteine; the content is the mass percentage of the mass of each component to the total mass of carbocisteine and each component;
the content of cystine is 0.04 %, the content of chloroacetic acid is 0.01 %, the content of carbocisteine lactam is 0.02 %, the content of *N*-carboxymethyl-*S*-carboxymethyl cysteine is 0.15 % and the content of impurity cysteine is 0, and the remaining content is carbocisteinee; the content is the mass percentage of the mass of each component to the total mass of carbocisteinee and each component;
the content of cystine is 0.01 %, the content of chloroacetic acid is 0, the content of carbocisteine lactam is 0, the content of *N*-carboxymethyl-*S*-carboxymethyl cysteine is 0.11 % and the content of impurity cysteine is 0, and the remaining content is carbocisteine; the content is the mass percentage of the mass of each component to the total mass of carbocisteine and each component;
the content of cystine is 0.02 %, the content of chloroacetic acid is 0, the content of carbocisteine lactam is 0, the content of *N*-carboxymethyl-*S*-carboxymethyl cysteine is 0.09 % and the content of impurity cysteine is 0, and the remaining content is carbocisteine; the content is the mass percentage of the mass of each component to the total mass of carbocisteine and each component;
the content of cystine is 0.05 %, the content of chloroacetic acid is 0, the content of carbocisteine lactam is 0, the content of *N*-carboxymethyl-*S*-carboxymethyl cysteine is 0.09 % and the content of impurity cysteine is 0, and the remaining content is carbocisteine; the content is the mass percentage of the mass of each component to the total mass of carbocisteine and each component;
the content of cystine is 0.05 %, the content of chloroacetic acid is 0, the content of carbocisteine lactam is 0, the content of *N*-carboxymethyl-*S*-carboxymethyl cysteine is 0.07 % and the content of impurity cysteine is 0.04 %, and the remaining content is carbocisteine; the content is the mass percentage of the mass of each component to the total mass of carbocisteine and each component;
the content of cystine is 0.04 %, the content of chloroacetic acid is 0.04 %, the content of carbocisteine lactam is 0, the content of *N*-carboxymethyl-*S*-carboxymethyl cysteine is 0.06 % and the content of impurity cysteine is 0.04 %, and the remaining content is carbocisteine; the content is the mass percentage of the mass of each component to the total mass of carbocisteine and each component;
the content of cystine is 0.07 %, the content of chloroacetic acid is 0.04 %, the content of carbocisteine lactam is 0, the content of *N*-carboxymethyl-*S*-carboxymethyl cysteine is 0.08 % and the content of impurity cysteine is 0.03 %, and the remaining content is carbocisteine; the content is the mass percentage of the mass of each component to the total mass of carbocisteine and each component;
the content of cystine is 0.09 %, the content of chloroacetic acid is 0, the content of carbocisteine lactam is 0.01 %, the content of *N*-carboxymethyl-*S*-carboxymethyl cysteine is 0.09 % and the content of impurity cysteine is 0, and the remaining content is carbocisteine; the content is the mass percentage of the mass of each component to the total mass of carbocisteine and each component;
and, the content of cystine is 0.1 %, the content of chloroacetic acid is 0, the content of carbocisteine lactam is 0.02 %, the content of *N*-carboxymethyl-*S*-carboxymethyl cysteine is 0.11 % and the content of impurity cysteine is 0, and the remaining content is carbocisteine; the content is the mass percentage of the mass of each component to the total mass of carbocisteine and each component.

In the present disclosure, "room temperature" refers to 10 to 30 °C.

On the basis of not violating the common sense in the art, the above-mentioned preferred conditions can be arbitrarily combined to obtain the preferred embodiments of the present disclosure.

The reagents and raw materials used in the present disclosure are commercially available.

The positive and progressive effect of the present disclosure is that when the continuous-flow reactor is used to prepare carbocisteine in the present disclosure, the content of related impurities (cystine, carbocisteine lactam, *N*-carboxymethyl-*S*-carboxymethyl cysteine, acetic acid and cysteine) in the reaction solution can be controlled at a low level, thereby a product that meets the requirements of CEP registration standards can be obtained through conventional post-treatment.

### DETAILED DESCRIPTION OF THE PREFERRED EMBODIMENT

The following embodiments further illustrate the present disclosure, but the present disclosure is not limited thereto. The experimental methods not specified in the specific conditions in the following embodiments are selected according to the conventional methods and conditions, or according to the commodity instructions.

The continuous-flow reactor used in the following embodiments is a micro-reactor (Floway F1 micro-reactor produced by Wuhan Shengshi Fine Chemicals Co., Ltd.). The micro-reactor uses five 3.3 high borosilicate glass plates, and the glass has a four-and three-cavity structure, with a reaction cavity in the middle and a heat exchange cavity on both sides. Each reaction plate has two feed ports and one discharge port. A single reaction plate has a volume of 10 mL, a cross section of 1.5 mm*1.5 mm, and a channel depth of about 1.2-1.5 mm. The micro-reaction equipment of the present disclosure is formed by inverse series connection of five plates, with a volume of 50 mL. The size of the reaction channel of the reactor is 1500 microns.

In the following embodiments, a continuous reaction is carried out through the micro-reactor. After the equipment is turned on, the material is continuously discharged, and a material liquid is collected at the discharge port. The reaction residence time may be changed by adjusting the flow rate, and the reaction temperature may be changed by a refrigeration heating temperature control system. In the meantime, a certain amount of the material liquid is collected for an HPLC detection, and the material liquid is crystallized and recrystallized to obtain a product.

The following embodiments use the Floway F1 micro-reactor produced by Wuhan Shengshi Fine Chemicals Co., Ltd. as an example only, and the same type of micro-reactors or similar micro-reactors produced by other manufacturers are also applicable to the present disclosure.

### Embodiment 1

Chloroacetic acid (II) and *L*-cysteine hydrochloride monohydrate (I) were respectively prepared into a mixed raw material liquid I-II at a molar ratio of 0.95:1, and 8 mol/L NaOH was prepared as a raw material liquid V, wherein the concentration of *L*-cysteine hydrochloride monohydrate was 1.25 mol/L, and the concentration of chloroacetic acid was 1.1875 mol/L. The feed concentration ratio of raw material liquids was I:II:V = 1 :0.95:3.2. The flow rate of the raw material liquid I-II was 4 mL/min, and the flow rate of the raw material liquid V was 2 mL/min. The reaction residence time was 8.33 min, and the reaction temperature was controlled at about 55±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise thereto and shaken well. The reaction solution showing blue indicated the end point of the reaction, and the reaction solution showing colorless indicated that the *Z-*cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was added with water to a constant volume to 7-10 times the weight of the crude product, heated to 50 to 55 °C. 8 mol/L NaOH was added dropwise thereto to adjust the pH to about 2.30. The mixture was cooled to 20 to 30 °C, filtered under reduced pressure, and dried to obtain 52.7 g of a fine product of carbocisteine with a yield of 90.1 %.

The HPLC related substance detection and chloride ion, specific rotation, loss on drying, residue on ignition, and content detection were respectively carried out on the obtained fine product. The detection results are shown in Table 2 and Table 3.

### Embodiment 2

Chloroacetic acid (II) and *L*-cysteine hydrochloride monohydrate (I) were respectively prepared into a mixed raw material liquid I-II at a molar ratio of 1:1, and 8 mol/L NaOH was prepared as a raw material liquid V, wherein the concentration of *L*-cysteine hydrochloride monohydrate was 1.25 mol/L, and the concentration of chloroacetic acid was 1.25 mol/L. The feed concentration ratio of raw material liquids was I:II:V = 1: 1:3.2. The flow rate of the raw material liquid I-II was 4 mL/min, and the flow rate of the raw material liquid V was 2 mL/min. The reaction residence time was 8.33 min, and the reaction temperature was controlled at about 55±1 °C. An end point was detected by sampling after the reaction. After the reaction, samples were taken for an HPLC related substance detection, and the detection results are shown in Table 1:

End point detection: 2 mL of reaction solution was taken, added with 5 mL of 2 mol/L HCl, and added with 2-3 drops of a 0.5 % starch solution. 1-3 drops of an I₂ solution were added dropwise thereto and shaken well. The reaction solution showing blue indicated the end point of the reaction, and the reaction solution showing colorless indicated that the *Z-*cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.07 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 52.86 g of a fine product of carbocisteine with a yield of 90.3 %.

The HPLC related substance detection and chloride ion, specific rotation, loss on drying, residue on ignition, and content detection were respectively carried out on the obtained fine product. The detection results are shown in Table 2 and Table 3.

### Embodiment 3

Chloroacetic acid (II) and *L*-cysteine hydrochloride monohydrate (I) were respectively prepared into a mixed raw material liquid I-II at a molar ratio of 1.02:1, and 8 mol/L NaOH was prepared as a raw material liquid V, wherein the concentration of *L*-cysteine hydrochloride monohydrate was 1.25 mol/L, and the concentration of chloroacetic acid was 1.275 mol/L. The feed concentration ratio of raw material liquids was I:II:V = 1: 1.02:3.2. The flow rate of the raw material liquid I-II was 4 mL/min, and the flow rate of the raw material liquid V was 2 mL/min. The reaction residence time was 8.33 min, and the reaction temperature was controlled at about 55±1 °C. An end point was detected by sampling after the reaction. After the reaction, samples were taken for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the L-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.38 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 55.09 g of a fine product of carbocisteine with a yield of 94.1 %.

The HPLC related substance detection and chloride ion, specific rotation, loss on drying, residue on ignition, and content detection were respectively carried out on the obtained fine product. The detection results are shown in Table 2 and Table 3.

### Embodiment 4

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.3125 mol/L. The feed concentration ratio of raw material liquids was I:II:V = 1:1.05:3.2. The flow rate of the raw material liquid I-II was 4 mL/min, and the flow rate of the raw material liquid V was 2 mL/min. The reaction residence time was 8.33 min, and the reaction temperature was controlled at about 55±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L-*cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.5 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 55.21 g of a fine product of carbocisteine with a yield of 94.3 %.

The HPLC related substance detection and chloride ion, specific rotation, loss on drying, residue on ignition, and content detection were respectively carried out on the obtained fine product. The detection results are shown in Table 2 and Table 3.

### Embodiment 5

Chloroacetic acid (II) and L-cysteine hydrochloride monohydrate (I) were respectively prepared into a mixed raw material liquid I-II at a molar ratio of 1.10:1, and 8 mol/L NaOH was prepared as a raw material liquid V, wherein the concentration of L-cysteine hydrochloride monohydrate was 1.25 mol/L, and the concentration of chloroacetic acid was 1.375 mol/L. The feed concentration ratio of raw material liquids was I:II:V = 1: 1.10:3.2. The flow rate of the raw material liquid I-II was 4 mL/min, and the flow rate of the raw material liquid V was 2 mL/min. The reaction residence time was 8.33 min, and the reaction temperature was controlled at about 55±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the L-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.08 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 54.8 g of a fine product of carbocisteine with a yield of 93.6 %.

The HPLC related substance detection and chloride ion, specific rotation, loss on drying, residue on ignition, and content detection were respectively carried out on the obtained fine product. The detection results are shown in Table 2 and Table 3.

### Embodiment 6

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.3125 mol/L. The feed concentration ratio of raw material liquids was I:II:V = 1:1.05:3.2. The flow rate of the raw material liquid I-II was 2 mL/min, and the flow rate of the raw material liquid V was 1 mL/min. The reaction residence time was 16.67 min, and the reaction temperature was controlled at about 55±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.1 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 54.9 g of a fine product of carbocisteine with a yield of 93.7 %.

The HPLC related substance detection and chloride ion, specific rotation, loss on drying, residue on ignition, and content detection were respectively carried out on the obtained fine product. The detection results are shown in Table 2 and Table 3.

### Embodiment 7

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.3125 mol/L. The feed concentration ratio of raw material liquids was I:II:V = 1:1.05:3.2. The flow rate of the raw material liquid I-II was 10 mL/min, and the flow rate of the raw material liquid V was 5 mL/min. The reaction residence time was 3.33 min, and the reaction temperature was controlled at about 55±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.6 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 55.3 g of a fine product of carbocisteine with a yield of 94.4 %.

The HPLC related substance detection and chloride ion, specific rotation, loss on drying, residue on ignition, and content detection were respectively carried out on the obtained fine product. The detection results are shown in Table 2 and Table 3.

### Embodiment 8

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.3125 mol/L. The feed concentration ratio of raw material liquids was I:II:V = 1:1.05:3.2. The flow rate of the raw material liquid I-II was 16 mL/min, and the flow rate of the raw material liquid V was 8 mL/min. The reaction residence time was 2.08 min, and the reaction temperature was controlled at about 55±1 °C. An end point was detected by sampling after the reaction. After the reaction, samples were taken for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 56.2 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 53.9 g of a fine product of carbocisteine with a yield of 92.1 %.

The HPLC related substance detection and chloride ion, specific rotation, loss on drying, residue on ignition, and content detection were respectively carried out on the obtained fine product. The detection results are shown in Table 2 and Table 3.

### Embodiment 9

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.275 mol/L. The feed concentration ratio of raw material liquids was I:II:V = 1:1.05:3.2. The flow rate of the raw material liquid I-II was 8 mL/min, and the flow rate of the raw material liquid V was 4 mL/min. The reaction residence time was 4.17 min, and the reaction temperature was controlled at about 35±1°C. An end point was detected by sampling after the reaction. After the reaction, samples were taken for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 54.9 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 52.7 g of a fine product of carbocisteine with a yield of 90.1 %.

The HPLC related substance detection and chloride ion, specific rotation, loss on drying, residue on ignition, and content detection were respectively carried out on the obtained fine product. The detection results are shown in Table 2 and Table 3.

### Embodiment 10

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.275 mol/L. The feed concentration ratio of raw material liquids was I:II:V = 1:1.05:3.2. The flow rate of the raw material liquid I-II was 8 mL/min, and the flow rate of the raw material liquid V was 4 mL/min. The reaction residence time was 4.17 min, and the reaction temperature was controlled at about 45±1 °C. An end point was detected by sampling after the reaction. After the reaction, sampling was carried out for an HPLC related substance detection, and the detection results are shown in Table 1:

End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.1 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 52.9 g of a fine product of carbocisteine with a yield of 90.3 %.

The HPLC related substance detection and chloride ion, specific rotation, loss on drying, residue on ignition, and content detection were respectively carried out on the obtained fine product. The detection results are shown in Table 2 and Table 3.

### Embodiment 11

Chloroacetic acid (II) and L-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.275 mol/L. The feed concentration ratio of raw material liquids was I:II:V = 1:1.05:3.2. The flow rate of the raw material liquid I-II was 8 mL/min, and the flow rate of the raw material liquid V was 4 mL/min. The reaction residence time was 4.17 min, and the reaction temperature was controlled at about 55±1 °C. An end point was detected by sampling after the reaction. After the reaction, sampling was carried out for an HPLC related substance detection, and the detection results are shown in Table 1:
End point detection: 2 mL of the reaction solution was taken, added with 5 mL of 2 mol/L HCl, and added with 2-3 drops of a 0.5 % starch solution. 1-3 drops of an I₂ solution were added dropwise and shaken well. The reaction solution showing blue indicated the end point of the reaction, and the reaction solution showing colorless indicated that the *L*-cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 57.1 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 54.9 g of a fine product of carbocisteine with a yield of 93.7 %.

The HPLC related substance detection and chloride ion, specific rotation, loss on drying, residue on ignition, and content detection were respectively carried out on the obtained fine product. The detection results are shown in Table 2 and Table 3.

### Embodiment 12

Chloroacetic acid (II) and L-cysteine hydrochloride monohydrate (I) were respectively prepared into a mixed raw material liquid I-II at a molar ratio of 1.05:1, and 8 mol/L NaOH was prepared as a raw material liquid V, wherein the concentration of L-cysteine hydrochloride monohydrate was 1.25 mol/L, and the concentration of chloroacetic acid was 1.275 mol/L. The feed concentration ratio of raw material liquids was I:II:V = 1:1.05:3.2. The flow rate of the raw material liquid I-II was 8 mL/min, and the flow rate of the raw material liquid V was 4 mL/min. The reaction residence time was 4.17 min, and the reaction temperature was controlled at about 65±1 °C. An end point was detected by sampling after the reaction. After the reaction, sampling was carried out for an HPLC related substance detection, and the detection results are shown in Table 1:

End point detection: 2 mL of reaction solution was taken, added with 5 mL of 2 mol/L HCl, and added with 2-3 drops of a 0.5 % starch solution. 1-3 drops of an I₂ solution were added dropwise thereto and shaken well. The reaction solution showing blue indicated the end point of the reaction, and the reaction solution showing colorless indicated that the *Z-*cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 56.9 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 54.6 g of a fine product of carbocisteine with a yield of 93.4 %.

The HPLC related substance detection and chloride ion, specific rotation, loss on drying, residue on ignition, and content detection were respectively carried out on the obtained fine product. The detection results are shown in Table 2 and Table 3.

### Embodiment 13

Chloroacetic acid (II) and *L*-cysteine hydrochloride monohydrate (I) were respectively prepared into a mixed raw material liquid I-II at a molar ratio of 1.05:1, and 8 mol/L NaOH was prepared as a raw material liquid V, wherein the concentration of *L*-cysteine hydrochloride monohydrate was 1.25 mol/L, and the concentration of chloroacetic acid was 1.275 mol/L. The feed concentration ratio of raw material liquids was I:II:V = 1:1.05:3.2. The flow rate of the raw material liquid I-II was 8 mL/min, and the flow rate of the raw material liquid V was 4 mL/min. The reaction residence time was 4.17 min, and the reaction temperature was controlled at about 75±1 °C. An end point was detected by sampling after the reaction. After the reaction, sampling was carried out for an HPLC related substance detection, and the detection results are shown in Table 1:

End point detection: 2 mL of reaction solution was taken, added with 5 mL of 2 mol/L HCl, and added with 2-3 drops of a 0.5 % starch solution. 1-3 drops of an I₂ solution were added dropwise thereto and shaken well. The reaction solution showing blue indicated the end point of the reaction, and the reaction solution showing colorless indicated that the *Z-*cysteine hydrochloride was not reacted completely.

400 mL of the reaction solution (the mass of *L*-cysteine hydrochloride monohydrate in the corresponding raw material was 58.54 g) was collected, and the reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.6 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was added with water to a constant volume to 7-10 times the weight of the crude product, heated to 50 to 55 °C. 8 mol/L NaOH was added dropwise thereto to adjust the pH to about 2.30. The mixture was cooled to 20 to 30 °C, filtered under reduced pressure, and dried to obtain 53.3 g of a fine product of carbocisteine with a yield of 91.1 %.

The HPLC related substance detection and chloride ion, specific rotation, loss on drying, residue on ignition, and content detection were respectively carried out on the obtained fine product. The detection results are shown in Table 2 and Table 3.

### Comparative embodiment 1

58.54 g of *L*-cysteine hydrochloride monohydrate and 32.0 g of chloroacetic acid were added into the reactor, wherein the molar ratio of *L*-cysteine hydrochloride monohydrate to chloroacetic acid was 1:1.02, and 120 mL of pure water was added to dissolve. 30 % NaOH was added dropwise thereto. In the entire dropwise addition process, the temperature was controlled within 60 °C and the time was controlled within 30 min. An end point was detected by sampling after the reaction. After the reaction, the reaction solution was adjusted to a constant volume of 400 mL, and sampling was carried out for HPLC related substance detection. The detection results are shown in Table 1.

The reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.6 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 52.7 g of a fine product of carbocisteine with a yield of 90.1 %.

### Comparative embodiment 2

58.54 g of *L*-cysteine hydrochloride monohydrate and 32.94 g of chloroacetic acid were added into the reactor, wherein the molar ratio of *L*-cysteine hydrochloride monohydrate to chloroacetic acid was 1:1.05, and 120 mL of pure water was added to dissolve. 30 % NaOH was added dropwise thereto. In the entire dropwise addition process, the temperature was controlled within 60 °C and the time was controlled within 30min. An end point was detected by sampling after the reaction. After the reaction, the reaction solution was adjusted to a constant volume of 400 mL, and sampling was carried out for HPLC related substance detection. The detection results are shown in Table 1.

The reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.6 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount *of L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 52.9 g of a fine product of carbocisteine with a yield of 90.4 %.

### Comparative embodiment 3

58.54 g of *L*-cysteine hydrochloride monohydrate and 34.5 g of chloroacetic acid were added into the reactor, wherein the molar ratio of *L*-cysteine hydrochloride monohydrate to chloroacetic acid was 1:1.1, and 120 mL of pure water was added to dissolve. 30 % NaOH was added dropwise thereto. In the entire dropwise addition process, the temperature was controlled within 60 °C and the time was controlled within 30 min. An end point was detected by sampling after the reaction. After the reaction, the reaction solution was adjusted to a constant volume of 400 mL, and sampling was carried out for HPLC related substance detection. The detection results are shown in Table 1.

The reaction solution was added with water to reach 410-580 mL, dissolved in water and heated to 60 °C, then the pH of the mixture was adjusted to 2.5-3.0 by adding with industrial hydrochloric acid, then the mixture was cooled to 20 to 35 °C by circulating water, filtered and washed with water to obtain 55.6 g of a crude product of carbocisteine.

6-7 times the volume of purified water was added to the obtained crude product, heated to about 50 to 55 °C. The industrial hydrochloric acid was added dropwise thereto until the crude product was completely dissolved. 1.5 % activated carbon (based on the amount of *L-*cysteine hydrochloride) was added thereto, decolorized and filtered. The filtrate was adjusted to 7-10 times the weight of the crude product by adding water, heated to 50 to 55 °C. 8 mol/L NaOH was added dropwise thereto to adjust the pH to about 2.30. The mixture was cooled to 20 to 30 °C, filtered under reduced pressure, and dried to obtain 53.9 g of a fine product of carbocisteine with a yield of 92.1 %.

### Effect embodiment 1

**Detection method:** High performance liquid chromatography (HPLC) method was used for the detection. Detection methods in Table 1 and Table 2 refer to "Separation and Identification of Impurities in Carbocisteine", Pharmaceutical analysis journal, Chin J Pharm Anal 2014, 34 (4) 707-710); and data in Table 3 are detected with reference to the detection method of related substances in JP 16.

**Detection instrument:** the model of high performance liquid chromatography (HPLC) is Hitachi L2420.

The contents of impurity cystine (hereinafter referred to as A), impurity chloroacetic acid (hereinafter referred to as B), impurity carbocisteine lactam (hereinafter referred to as C), impurity N-carboxymethyl-S-carboxymethyl cysteine (hereinafter referred to as D) and impurity cysteine (hereinafter referred to as Cys) in the reaction solutions obtained in embodiments 1-13 and comparative embodiments 1-3 were detected. The specific detection results are shown in Table 1.

The contents of cystine, chloroacetic acid, carbocisteine lactam, *N*-carboxymethyl-*S-*carboxymethyl cysteine and cysteine in the fine products of carbocisteine obtained in embodiments 1-13 and comparative embodiments 1-3 were detected. The specific detection results are shown in Table 2.

The yield and chloride, specific rotation, loss on drying, residue on ignition, content detection results of the fine products of carbocisteine obtained in embodiments 1-13 and comparative embodiments 1-3 are shown in Table 3.

**Table 1**

| Embodiment | Molar ratio of chloroacetic acid to *L-*cysteine hydrochloride monohydrate | Residence time /min | Temperature/°C | A/mg/ mL | B/mg/ mL | C/mg/ mL | D/mg/ mL | Cys/mg/m L |
|---|---|---|---|---|---|---|---|---|
| Embodiment 1 | 0.95:1 | 8.33 | 55±1 | 0.11 | 0 | 0 | 0.42 | 6.44 |
| Embodiment 2 | 1.00:1 | 8.33 | 55±1 | 0.09 | 0 | 0.02 | 0.66 | 1.12 |
| Embodiment 3 | 1.02:1 | 8.33 | 55±1 | 0.13 | 1.95 | 0.02 | 0.7 | 0.68 |
| Embodiment 4 | 1.05:1 | 8.33 | 55±1 | 0.13 | 2.79 | 0.01 | 1.03 | 0 |
| Embodiment 5 | 1.10:1 | 8.33 | 55±1 | 0.11 | 4.02 | 0.02 | 1.51 | 0 |
| Embodiment 6 | 1.05:1 | 16.67 | 55±1 | 0.11 | 2.57 | 0.02 | 1.34 | 0 |
| Embodiment 7 | 1.05:1 | 3.33 | 55±1 | 0.14 | 2.88 | 0.02 | 1.09 | 0 |
| Embodiment 8 | 1.05:1 | 2.08 | 55±1 | 0.11 | 3.96 | 0.01 | 0.83 | 0.58 |
| Embodiment 9 | 1.02:1 | 4.17 | 35±1 | 0.2 | 6.64 | 0 | 0.22 | 2.4 |
| Embodiment 10 | 1.02:1 | 4.17 | 45±1 | 0.17 | 2.96 | 0 | 0.33 | 0.94 |
| Embodiment 11 | 1.02:1 | 4.17 | 55±1 | 0.17 | 2.31 | 0.02 | 0.54 | 0.8 |
| Embodiment 12 | 1.02:1 | 4.17 | 65±1 | 0.11 | 0.97 | 0.02 | 0.69 | 0.55 |
| Embodiment 13 | 1.02:1 | 4.17 | 75±1 | 0.13 | 0.95 | 0.03 | 0.82 | 0.45 |
| Comparative Embodiment 1 | 1.02:1 | \ | 55±5 | 0.29 | 0.82 | 0.08 | 1.50 | 2.4 |
| Comparative Embodiment 2 | 1.05:1 | \ | 55±5 | 0.28 | 0.91 | 0.11 | 1.91 | 2.21 |
| Comparative Embodiment 3 | 1.1:1 | \ | 55±5 | 0.34 | 1.02 | 0.12 | 1.94 | 0.55 |

In Table 1, the contents of impurity A, impurity C and impurity D in the carbocisteine reaction solution obtained by the preparation method of the present disclosure are significantly lower than those of comparative embodiments 1-3, or some of the contents are equivalent.

As can be seen from Table 1, when the molar ratio of chloroacetic acid to *L*-cysteine hydrochloride monohydrate is 0.95:1-1.05:1, the content of impurity D is low, and this range better reduces the generation of impurity D. When the residence time is 2.08-3.33min, the content of impurity D is low. When the temperature of the condensation reaction is 35±1 to 65±1°C, the contents of impurities C and D are both low.

**Table 2**

| | A/ % | B/ % | C/ % | D/ % | Cys/ % |
|---|---|---|---|---|---|
| CEP standard | ≤ 0.2 | ≤0.05 | ≤0.05 | ≤0.2 | ≤0.05 |
| Embodiment 1 | 0.03 | 0 | 0 | 0.03 | 0.87 |
| Embodiment 2 | 0.05 | 0 | 0 | 0.1 | 0.04 |
| Embodiment 3 | 0.06 | 0 | 0 | 0.12 | 0.01 |
| Embodiment 4 | 0.05 | 0 | 0 | 0.12 | 0 |
| Embodiment 5 | 0.04 | 0.01 | 0.02 | 0.15 | 0 |
| Embodiment 6 | 0.01 | 0 | 0 | 0.11 | 0 |
| Embodiment 7 | 0.02 | 0 | 0 | 0.09 | 0 |
| Embodiment 8 | 0.05 | 0 | 0 | 0.09 | 0 |
| Embodiment 9 | 0.05 | 0 | 0 | 0.07 | 0.04 |
| Embodiment 10 | 0.04 | 0.04 | 0 | 0.06 | 0.04 |
| Embodiment 11 | 0.07 | 0.04 | 0 | 0.08 | 0.03 |
| Embodiment 12 | 0.09 | 0 | 0.01 | 0.09 | 0 |
| Embodiment 13 | 0.1 | 0 | 0.02 | 0.11 | 0 |
| Comparative Embodiment 1 | 0.12 | 0.04 | 0.02 | 0.16 | 0.13 |
| Comparative Embodiment 2 | 0.15 | 0.03 | 0.03 | 0.19 | 0.11 |
| Comparative Embodiment 3 | 0.18 | 0.02 | 0.01 | 0.20 | 0.04 |

**Table 3**

| | Yield/ % | Chloride/pp m | Optical rotation | Loss on drying | Residue on ignition | Content/ % |
|---|---|---|---|---|---|---|
| JP-16 | | <710 | -33.5 to - 36.5 | <0.3 % | <0.1 % | >98.5 % |
| Embodiment 1 | 90.1 | <200 | -34.4 | 0.04 | 0.08 | 98.7 |
| Embodiment 2 | 90.3 | <200 | -34.3 | 0.03 | 0.08 | 98.8 |
| Embodiment 3 | 94.1 | <200 | -34.8 | 0.02 | 0.06 | 99.8 |
| Embodiment 4 | 94.3 | <200 | -34.2 | 0.01 | 0.06 | 99.5 |
| Embodiment 5 | 93.6 | <200 | -34.2 | 0.04 | 0.08 | 99.6 |
| Embodiment 6 | 93.7 | <200 | -34.1 | 0.03 | 0.08 | 99.5 |
| Embodiment 7 | 94.4 | <200 | -34.5 | 0.03 | 0.06 | 99.7 |
| Embodiment 8 | 92.1 | <200 | -34.4 | 0.05 | 0.07 | 99.2 |
| Embodiment 9 | 90.1 | <200 | -34.3 | 0.07 | 0.07 | 99.5 |
| Embodiment 10 | 90.3 | <200 | -34.0 | 0.04 | 0.08 | 99.1 |
| Embodiment 12 | 93.7 | <200 | -34.3 | 0.03 | 0.05 | 99.8 |
| Embodiment 12 | 93.4 | <200 | -34.6 | 0.02 | 0.08 | 99.2 |
| Embodiment 13 | 91.1 | <200 | -34.7 | 0.03 | 0.08 | 99.4 |
| Comparative Embodiment 1 | 90.1 | <200 | -34.5 | 0.04 | 0.08 | 99.4 |
| Comparative Embodiment 2 | 90.4 | <200 | -34.6 | 0.03 | 0.09 | 99.1 |
| Comparative Embodiment 3 | 92.1 | <200 | -34.2 | 0.07 | 0.08 | 99.7 |

Those skilled in the art know that the product of the present disclosure is difficult to separate from some impurities, such as cystine. However, the inventor of the present disclosure has controlled the above-mentioned impurities in a relatively low range through a large number of experiments and the method of the present disclosure, thus merely requiring a conventional post-treatment to obtain qualified products.

It can be seen from the data in Table 2 that in the detection results of the reaction solution (Table 1), the content of each impurity in comparative embodiments 1-3 is not well controlled, which leads to the difficulty in obtaining the product that meets the CEP registration standards after the same conventional purification post-treatment; however, the data in the present disclosure have certain advantages, thus ensuring that the product that meets the CEP registration standards can be obtained after the conventional purification post-treatment.

Although the specific embodiments of the present disclosure have been described above, those skilled in the art should understand that these are only examples, various changes or modifications can be made to these embodiments without departing from the principle and essence of the present disclosure. Therefore, the protection scope of the present disclosure is defined by the appended claims.

## Claims

1. A method for preparing carbocisteine, wherein the method comprises the following steps: in a continuous-flow reactor, carrying out a condensation reaction as shown below between *L*-cysteine as shown in formula I and a compound as shown in formula II in the presence of a base and in a solvent to obtain the carbocisteine as shown in formula III;
wherein, the size of a reaction channel of the continuous-flow reactor is 0.01-200 mm; a molar ratio of the compound as shown in formula II to the *L*-cysteine as shown in formula I is 0.95:1-1.10:1; the temperature of the condensation reaction is 35±1 to 75±1 °C; a residence time of the condensation reaction is 2.08-16.67 min; 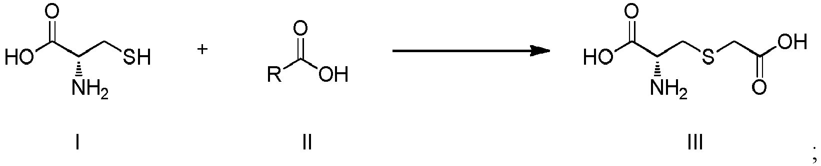
R is methyl substituted by halogen.

2. The method for preparing as claimed in claim 1, wherein, in the condensation reaction, a molar ratio of the base to the *L*-cysteine as shown in formula I is 3:1-4:1;
and/or, in the condensation reaction, a molar ratio of the compound as shown in formula II to the *L*-cysteine as shown in formula I is 0.95:1-1.05:1;
and/or, the temperature of the condensation reaction is 35±1 to 65±1 °C;
and/or, the time of the condensation reaction is 2.08-3.33 min;
and/or, a reagent used in the condensation reaction does not include one or more of Na₂CO₃, NaHCO₃, K₂CO₃ and KHCO₃;
and/or, the continuous-flow reactor is a micro-reactor or a tubular reactor;
and/or, in formula II, when R is the methyl substituted by halogen, the halogen is F, Cl, Br or I;
and/or, in formula II, when R is the methyl substituted by halogen, the number of the substitutions is 1;
and/or, in the condensation reaction, the base is an alkali metal base and/or an ammonia solution;
and/or, in the condensation reaction, the solvent is water;
and/or, in the condensation reaction, the *L*-cysteine as shown in formula I is a salt of *L-*cysteine and/or a *L*-cysteine hydrochloride monohydrate;
and/or, in the condensation reaction, the compound as shown in formula II is chloroacetic acid.

3. The method for preparing as claimed in claim 2, wherein, the molar ratio of the compound as shown in formula II to the *L*-cysteine as shown in formula I is 1.00: 1-1.05: 1;
and/or, the temperature of the condensation reaction is 35±1 to 55±1 °C;
and/or, the reagent used in the condensation reaction does not include one or more of Na₂SO₃ and/or Na₂S₂O₅;
and/or, the continuous-flow reactor is the micro-reactor;
and/or, in the condensation reaction, in formula II, when R is the methyl substituted by halogen, the halogen is Cl;
and/or, in the condensation reaction, the base is the alkali metal base;
and/or, in the condensation reaction, the *L*-cysteine as shown in formula I is the *L*-cysteine hydrochloride monohydrate;
and/or, in the condensation reaction, the *L*-cysteine as shown in the formula I is introduced into the micro-reactor in the form of a solution to carry out the condensation reaction.

4. The method for preparing as claimed in claim 3, wherein, when the continuous-flow reactor is the micro-reactor, the micro-reactor is a Corning micro-reactor LF, Wenhao WH-IND MT280 or Wuhan Shengshi Fine Chemicals Floway F1; the micro-reactor may also be Wuhan Shengshi Fine Chemicals Floway F1;
and/or, in the condensation reaction, when the base is the alkali metal base, the alkali metal base is sodium hydroxide;
and/or, in the condensation reaction, the compound as shown in formula II is chloroacetic acid.

5. The method for preparing as claimed in claim 1, wherein, in the condensation reaction, the base is introduced into the continuous-flow reactor in the form of a solution to carry out the condensation reaction;
and/or, in the condensation reaction, the *L*-cysteine as shown in the formula I is introduced into the continuous-flow reactor in the form of a solution to carry out the condensation reaction;
and/or, in the condensation reaction, the compound as shown in formula II is introduced into the continuous-flow reactor in the form of a solution to carry out the condensation reaction.

6. The method for preparing as claimed in claim 5, wherein, when the base is introduced into the continuous-flow reactor in the form of the solution, a flow rate of the solution is 1-8 mL/min;
and/or, when the base is introduced into the continuous-flow reactor in the form of the solution, a molar concentration of the base is 6-9 mol/L;
and/or, when the *L*-cysteine as shown in the formula I is introduced into the continuous-flow reactor in the form of the solution, a flow rate of the solution is 2-16 mL/min;
and/or, when the *L*-cysteine as shown in formula I is introduced into the continuous-flow reactor in the form of the solution, a molar concentration of the *L*-cysteine as shown in formula I is 1-3 mol/L;
and/or, when the compound as shown in formula II is introduced into the continuous-flow reactor in the form of the solution, a flow rate of the solution is 2-16 mL/min;
and/or, when the compound as shown in formula II is introduced into the continuous-flow reactor in the form of the solution, a molar concentration of the compound as shown in formula II is 1.1875-1.3125 mol/L.

7. The method for preparing as claimed in claim 6, wherein, when the *L*-cysteine as shown in the formula I is introduced into the continuous-flow reactor in the form of the solution, the flow rate of the solution is 10-16 mL/min;
and/or, when the compound as shown in formula II is introduced into the continuous-flow reactor in the form of the solution, the flow rate of the solution is 10-16 mL/min.

8. The method for preparing as claimed in at least one of claims 1 to 7, wherein, mixing the *L*-cysteine as shown in formula I, the compound as shown in formula II and the solvent to obtain a solution I-II, mixing the base and the solvent to obtain a solution V, and pumping the solution I-II and the solution V into the continuous-flow reactor respectively; wherein, the continuous-flow reactor is the micro-reactor.

9. The method for preparing as claimed in at least one of claims 1 to 8, wherein, the method for preparing comprises the following steps: mixing the *L*-cysteine as shown in formula I, the compound as shown in formula II and the solvent to obtain the solution I-II, mixing the base and the solvent to obtain the solution V, pumping the solution I-II and the solution V into the continuous-flow reactor respectively, setting a reaction temperature, controlling the flow rates of the solution I-II and the solution V, controlling the flow rate ratio of the solution I-II to the solution V, and completing the reaction within the reaction residence time; wherein, the continuous-flow reactor is the micro-reactor; the molar ratio of the *L*-cysteine as shown in formula I to the compound as shown in formula II, the reaction temperature, the flow rates of the solution I-II and the solution V, the flow rate ratio and the residence time are all as described in at least one of claims 1 to 8.

10. The method for preparing as claimed in at least one of claims 1 to 9, wherein, a post-treatment of the condensation reaction comprises the following steps: diluting, adjusting the pH value to 2.5-3.0, cooling, recrystallizing, filtering and drying.

11. The method for preparing as claimed in claim 10, wherein, in the step of diluting, a solvent used for diluting is water; and/or, a volume mass ratio of the solvent for diluting to the *L*-cysteine as shown in formula I is 7-10 mL/g;
and/or, in the step of adjusting the pH value to 2.5-3.0, a method for adjusting the pH value to 2.5-3.0 is to use a regulator to adjust; the regulator is preferably inorganic acid, further preferably hydrochloric acid;
and/or, before the step of adjusting the pH value to 2.5-3.0, a reaction solution after diluting is heated to 50 to 70 °C;
and/or, in the step of cooling, a temperature for cooling is 20 to 35 °C;
and/or, in the step of recrystallizing, a solvent used for recrystallizing is water; and/or, a volume mass ratio of the solvent used for recrystallizing to the crude product of the carbocisteine is 6-7 mL/g; and/or, a temperature for recrystallizing is 50 to 55 °C; and/or, the recrystallizing further comprises a decolorizing step; preferably, after the step of decolorizing, a pH value of the recrystallized solution is adjusted to 2-2.5.

## Patentansprüche

1. Herstellungsverfahren von Carbocistein, wobei das Verfahren die folgenden Schritte umfasst: in einem Durchlaufreaktor, Durchführen einer Kondensationsreaktion, wie unten gezeigt, zwischen Z-Cystein, wie gezeigt in Formel I, und einer Verbindung, wie gezeigt in Formel II, in Anwesenheit einer Base und in einem Lösungsmittel, um das Carbocistein, wie gezeigt in Formel III, zu erlangen;
wobei die Größe eines Reaktionskanals des Durchlaufreaktors 0,01-200 mm ist; ein Molverhältnis der Verbindung, wie gezeigt in Formel II, zu dem Z-Cystein, wie gezeigt in Formel I, 0,95:1-1,10:1 ist; die Temperatur der Kondensationsreaktion 35±1 bis 75±1 °C ist; die Verweilzeit der Kondensationsreaktion 2,08-16,67 min ist; 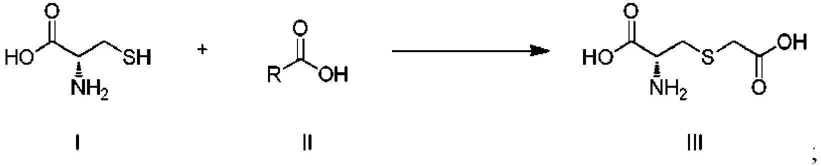
R durch Halogen substituiertes Methyl ist.

2. Herstellungsverfahren nach Anspruch 1, wobei bei der Kondensationsreaktion das molare Verhältnis der Base zu dem in Formel I gezeigten Z-Cystein 3:1-4:1 ist;
und/oder bei der Kondensationsreaktion das molare Verhältnis der in Formel II gezeigten Verbindung zu dem in Formel I gezeigten Z-Cystein 0,95:1-1,05:1 ist;
und/oder die Temperatur der Kondensationsreaktion 35±1 bis 65±1 °C ist;
und/oder die Dauer der Kondensationsreaktion 2,08-3,33 Minuten ist;
und/oder ein Reagenz, das in der Kondensationsreaktion verwendet, eines oder mehrere von Na₂CO₃, NaHCO₃, K₂CO₃ und KHCO₃ nicht beinhaltet;
und/oder der Durchlaufreaktor ein Mikroreaktor oder ein Röhrenreaktor ist;
und/oder wenn in der Formel II R eine durch Halogen substituierte Methylgruppe ist, das Halogen F, Cl, Br oder I ist;
und/oder wenn in Formel II R eine durch Halogen substituierte Methylgruppe ist, die Anzahl der Substitutionen 1 ist;
und/oder bei der Kondensationsreaktion die Base eine Alkalimetallbase und/oder eine Ammoniaklösung ist;
und/oder bei der Kondensationsreaktion das Lösungsmittel Wasser ist;
und/oder bei der Kondensationsreaktion das in Formel I gezeigte Z-Cystein ein Salz von Z-Cystein und/oder ein Z-Cysteinhydrochlorid-Monohydrat ist;
und/oder bei der Kondensationsreaktion die in Formel II dargestellte Verbindung Chloressigsäure ist.

3. Herstellungsverfahren nach Anspruch 2, wobei das molare Verhältnis der in Formel II gezeigten Verbindung zu dem in Formel I gezeigten Z-Cystein 1,00:1-1,05:1 ist;
und/oder die Temperatur der Kondensationsreaktion 35±1 bis 55±1 °C ist;
und/oder das in der Kondensationsreaktion verwendete Reagenz nicht eines oder mehrere von Na₂SO₃ und/oder Na₂S₂O₅ beinhaltet;
und/oder der Durchlaufreaktor der Mikroreaktor ist;
und/oder wenn bei der Kondensationsreaktion in der Formel II R das durch Halogen substituierte Methyl ist, das Halogen Cl ist;
und/oder bei der Kondensationsreaktion die Base eine Alkalimetallbase ist;
und/oder bei der Kondensationsreaktion das in Formel I gezeigte Z-Cystein das Z-Cysteinhydrochlorid-Monohydrat ist;
und/oder bei der Kondensationsreaktion das in der Formel I gezeigte Z-Cystein in Form einer Lösung in den Mikroreaktor eingebracht wird, um die Kondensationsreaktion durchzuführen.

4. Herstellungsverfahren nach Anspruch 3, wobei, wenn der Durchlaufreaktor der Mikroreaktor ist, der Mikroreaktor ein Corning Mikroreaktor LF, Wenhao WH-IND MT280 oder Wuhan Shengshi Fine Chemicals Floway Fl ist; der Mikroreaktor kann auch Wuhan Shengshi Fine Chemicals Floway F1 sein;
und/oder wenn bei der Kondensationsreaktion die Base eine Alkalimetallbase ist, die Alkalimetallbase Natriumhydroxid ist;
und/oder bei der Kondensationsreaktion die in Formel II gezeigte Verbindung Chloressigsäure ist.

5. Herstellungsverfahren nach Anspruch 1, **dadurch gekennzeichnet, dass** bei der Kondensationsreaktion die Base in Form einer Lösung in den Durchlaufreaktor eingebracht wird, um die Kondensationsreaktion durchzuführen;
und/oder bei der Kondensationsreaktion das in der Formel I gezeigte Z-Cystein in Form einer Lösung in den Durchlaufreaktor eingebracht, um die Kondensationsreaktion durchzuführen;
und/oder bei der Kondensationsreaktion die in Formel II gezeigte Verbindung in Form einer Lösung in den Durchlaufreaktor eingebracht wird, um die Kondensationsreaktion durchzuführen.

6. Herstellungsverfahren nach Anspruch 5, wobei, wenn die Base in Form der Lösung in den Durchlaufreaktor eingebracht wird, eine Durchflussrate der Lösung 1-8 ml/Min ist;
und/oder, wenn die Base in Form der Lösung in den Durchlaufreaktor eingebracht wird, eine molare Konzentration der Base 6-9 mol/l ist;
und/oder, wenn das in der Formel I gezeigte Z-Cystein in Form der Lösung in den Durchlaufreaktor eingebracht wird, eine Durchflussrate der Lösung 2-16 ml/Min ist;
und/oder wenn das in der Formel I gezeigte Z-Cystein in Form der Lösung in den Durchlaufreaktor eingebracht wird, die molare Konzentration des in Formel I gezeigten Z-Cysteins 1-3 mol/l ist;
und/oder wenn die in Formel II gezeigte Verbindung in Form der Lösung in den Durchlaufreaktor eingebracht wird, eine Durchflussrate der Lösung 2-16 ml/Min ist;
und/oder wenn die in Formel II gezeigte Verbindung in Form der Lösung in den Durchlaufreaktor eingebracht wird, die molare Konzentration der in Formel II gezeigten Verbindung 1,1875-1,3125 mol/l ist.

7. Herstellungsverfahren nach Anspruch 6, **dadurch gekennzeichnet, dass**, wenn das in der Formel I gezeigte Z-Cystein in Form der Lösung in den Durchlaufreaktor eingebracht wird, die Durchflussrate der Lösung 10-16 ml/Min ist;
und/oder wenn die in Formel II gezeigte Verbindung in Form der Lösung in den Durchlaufreaktor eingebracht wird, die Durchflussrate der Lösung 10-16 ml/Min ist.

8. Herstellungsverfahren gemäß mindestens einem der Ansprüche 1 bis 7, wobei das in Formel I gezeigte Z-Cystein, die in Formel II gezeigte Verbindung und das Lösungsmittel gemischt werden, um eine Lösung I-II zu erlangen, die Base und das Lösungsmittel gemischt werden, um eine Lösung V zu erlangen, und die Lösung I-II und die Lösung V in den Durchlaufreaktor gepumpt werden; wobei der Durchlaufreaktor der Mikroreaktor ist.

9. Herstellungsverfahren wie in mindestens einem der Ansprüche 1 bis 8 beansprucht, wobei das Herstellungsverfahren die folgenden Schritte umfasst: Mischen des in Formel I gezeigten Z-Cysteins, der in Formel II gezeigten Verbindung und des Lösungsmittels, um die Lösung I-II zu erlangen, Mischen der Base und des Lösungsmittels, um die Lösung V zu erlangen, Pumpen der Lösung I-II bzw. der Lösung V in den Durchlaufreaktor, Einstellen einer Reaktionstemperatur, Steuern der Durchflussmengen der Lösungen I-II und der Lösung V, Steuern des Durchflussmengenverhältnisses der Lösungen I-II und der Lösung V, und Vervollständigen der Reaktion innerhalb der Reaktionsverweilzeit; wobei der Durchlaufreaktor der Mikroreaktor ist; das molare Verhältnis des in Formel I gezeigten Z-Cysteins zu der in Formel II gezeigten Verbindung, die Reaktionstemperatur, die Durchflussmengen der Lösungen I-II und der Lösung V, das Durchflussmengenverhältnis und die Verweilzeit alle wie beschrieben in mindestens einem der Ansprüche 1 bis 8 sind.

10. Herstellungsverfahren nach mindestens einem der Ansprüche 1 bis 9, wobei eine Nachbehandlung der Kondensationsreaktion die folgenden Schritte umfasst: Verdünnen, Einstellen des pH-Werts auf 2,5-3,0, Abkühlen, Umkristallisieren, Filtrieren und Trocknen.

11. Herstellungsverfahren nach Anspruch 10, wobei im Schritt eines Verdünnens ein zum Verdünnen verwendetes Lösungsmittel Wasser ist; und/oder ein Volumenmassenverhältnis des Lösungsmittels zum Verdünnen zu dem in Formel I gezeigten Z-Cystein 7-10 ml/g ist;
und/oder im Schritt eines Einstellen des pH-Werts auf 2,5-3,0 ein Verfahren zum Einstellen des pH-Werts auf 2,5-3,0 in der Verwendung eines Einstellungsregulators besteht; wobei der Regulator vorzugsweise anorganische Säure, bevorzugter Salzsäure ist;
und/oder vor dem Schritt eines Einstellens des pH-Werts auf 2,5-3,0 die Reaktionslösung nach Verdünnen auf 50 bis 70 °C erhitzt wird;
und/oder im Schritt eines Abkühlens eine Kühltemperatur 20 bis 35 °C ist;
und/oder im Schritt eines Umkristallisierens ein zum Umkristallisieren verwendetes Lösungsmittel Wasser ist; und/oder ein Volumenmassenverhältnis des zum Umkristallisieren verwendeten Lösungsmittels zu dem Rohprodukt des Carbocisteins 6-7 ml/g ist; und/oder eine Temperatur zum Umkristallisieren 50 bis 55 °C ist; und/oder das Umkristallisieren ferner einen Entfärbungsschritt umfasst; wobei vorzugsweise nach dem Schritt eines Entfärbens ein pH-Wert der umkristallisierten Lösung auf 2-2,5 eingestellt wird.

## Revendications

1. Procédé permettant la préparation de la carbocystéine, ledit procédé comprenant les étapes suivantes : dans un réacteur à écoulement continu, la réalisation d'une réaction de condensation telle que présentée ci-dessous entre la *L*-cystéine telle que présentée dans la formule I et un composé tel que présenté dans la formule II en présence d'une base et dans un solvant pour obtenir la carbocystéine telle que présentée dans la formule III ;
la taille d'un canal de réaction du réacteur à écoulement continu valant de 0,01 à 200 mm ; un rapport molaire du composé tel que présenté dans la formule II à la *L*-cystéine telle que présentée dans la formule I valant de 0,95:1 à 1,10:1 ; la température de la réaction de condensation étant de 35±1 à 75±1°C ; un temps de séjour de la réaction de condensation étant de 2,08 à 16,67 min ; 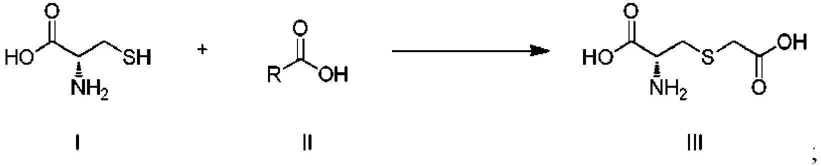
R représentant un groupe méthyle substitué par un atome d'halogène.

2. Procédé permettant la préparation selon la revendication 1, dans la réaction de condensation, un rapport molaire de la base à la *L*-cystéine telle que présentée dans la formule I valant de 3:1 à 4:1 ;
et/ou, dans la réaction de condensation, ledit rapport molaire du composé tel que présenté dans la formule II à la *L*-cystéine telle que présentée dans la formule I valant de 0.95: 1 à 1.05: 1 ;
et/ou ladite température de la réaction de condensation valant de 35±1 à 65±1°C ;
et/ou la durée de la réaction de condensation valant de 2,08 à 3,33 min ;
et/ou le réactif utilisé dans la réaction de condensation ne comprenant pas un ou plusieurs composés parmi Na₂CO₃, NaHCO₃, K₂CO₃ et KHCO₃ ;
et/ou ledit réacteur à écoulement continu étant un micro-réacteur ou un réacteur tubulaire ;
et/ou, dans la formule II, lorsque R représente le groupe méthyle substitué par un atome d'halogène, ledit atome d'halogène étant F, Cl, Br ou I ;
et/ou, dans la formule II, lorsque R représente le groupe méthyle substitué par un atome d'halogène, le nombre des substitutions étant de 1 ;
et/ou, dans la réaction de condensation, ladite base étant une base de métal alcalin et/ou une solution d'ammoniaque ;
et/ou, dans la réaction de condensation, ledit solvant étant l'eau ;
et/ou, dans la réaction de condensation, ladite *L*-cystéine telle que présentée dans la formule I étant un sel de *L*-cystéine et/ou un chlorhydrate de *L*-cystéine monohydraté ;
et/ou, dans la réaction de condensation, ledit composé tel que présenté dans la formule II étant l'acide chloroacétique.

3. Procédé permettant la préparation selon la revendication 2, ledit rapport molaire du composé tel que présenté dans la formule II à la *L*-cystéine telle que présentée dans la formule I valant de 1,00:1 à 1,05:1 ;
et/ou ladite température de la réaction de condensation étant de 35±1 à 55±1°C ;
et/ou ledit réactif utilisé dans la réaction de condensation ne comprenant pas un ou plusieurs composés parmi Na₂SO₃ et/ou Na₂S₂O₅ ;
et/ou ledit réacteur à écoulement continu étant le micro-réacteur ;
et/ou, dans la réaction de condensation, dans la formule II, lorsque R représente le groupe méthyle substitué par un atome d'halogène, ledit atome d'halogène étant Cl ;
et/ou, dans la réaction de condensation, ladite base étant la base de métal alcalin ;
et/ou, dans la réaction de condensation, ladite *L*-cystéine telle que présentée dans la formule I étant le chlorhydrate de *L*-cystéine monohydraté ;
et/ou, dans la réaction de condensation, ladite *L*-cystéine telle que présentée dans la formule I étant introduite dans le micro-réacteur sous la forme d'une solution pour réaliser la réaction de condensation.

4. Procédé permettant la préparation selon la revendication 3, lorsque le réacteur à écoulement continu est le micro-réacteur, ledit micro-réacteur étant un micro-réacteur LF de Corning, WH-IND MT280 de Wenhao ou Floway Fl de Wuhan Shengshi Fine Chemicals ; ledit micro-réacteur pouvant également être le Floway Fl de Wuhan Shengshi Fine Chemicals ;
et/ou, dans la réaction de condensation, lorsque la base est la base de métal alcalin, ladite base de métal alcalin étant l'hydroxyde de sodium ;
et/ou, dans la réaction de condensation, ledit composé tel que présenté dans la formule II étant l'acide chloroacétique.

5. Procédé permettant la préparation selon la revendication 1, dans la réaction de condensation, ladite base étant introduite dans le réacteur à écoulement continu sous la forme d'une solution pour réaliser la réaction de condensation ;
et/ou, dans la réaction de condensation, ladite *L*-cystéine telle que présentée dans la formule I étant introduite dans le réacteur à écoulement continu sous la forme d'une solution pour réaliser la réaction de condensation ;
et/ou, dans la réaction de condensation, ledit composé tel que présenté dans la formule II étant introduit dans le réacteur à écoulement continu sous la forme d'une solution pour réaliser la réaction de condensation.

6. Procédé permettant la préparation selon la revendication 5, lorsque la base est introduite dans le réacteur à écoulement continu sous la forme de la solution, un débit de la solution valant de 1 à 8 ml/min ;
et/ou, lorsque la base est introduite dans le réacteur à écoulement continu sous la forme de la solution, une concentration molaire de la base valant de 6 à 9 mol/l ;
et/ou, lorsque la *L*-cystéine telle que présentée dans la formule I est introduite dans le réacteur à écoulement continu sous la forme de la solution, un débit de la solution valant de 2 à 16 ml/min ;
et/ou, lorsque la *L*-cystéine telle que présentée dans la formule I est introduite dans le réacteur à écoulement continu sous la forme de la solution, une concentration molaire de la *L-*cystéine telle que présentée dans la formule I valant de 1 à 3 mol/l ;
et/ou, lorsque le composé telle que présentée dans la formule II est introduit dans le réacteur à écoulement continu sous la forme de la solution, un débit de la solution valant de 2 à 16 ml/min ;
et/ou, lorsque le composé telle que présentée dans la formule II est introduit dans le réacteur à écoulement continu sous la forme de la solution, une concentration molaire du composé tel que présenté dans la formule II valant de 1,1875 à 1,3125 mol/l.

7. Procédé permettant la préparation selon la revendication 6, lorsque la *L*-cystéine telle que présentée dans la formule I est introduite dans le réacteur à écoulement continu sous la forme de la solution, ledit débit de la solution valant de 10 à 16 ml/min ;
et/ou, lorsque le composé telle que présenté dans la formule II est introduit dans le réacteur à écoulement continu sous la forme de la solution, ledit débit de la solution valant de 10 à 16 ml/min.

8. Procédé permettant la préparation selon au moins l'une des revendications 1 à 7, le mélange de la *L*-cystéine telle que présentée dans la formule I, du composé tel que présenté dans la formule II et du solvant pour obtenir une solution I-II, le mélange de la base et du solvant pour obtenir une solution V et le pompage de la solution I-II et de la solution V dans le réacteur à écoulement continu, respectivement ; ledit réacteur à écoulement continu étant le micro-réacteur.

9. Procédé permettant la préparation selon au moins l'une des revendications 1 à 8, ledit procédé de préparation comprenant les étapes suivantes : le mélange de la *L*-cystéine telle que présentée dans la formule I, du composé tel que présenté dans la formule II et du solvant pour obtenir la solution I-II, le mélange de la base et du solvant pour obtenir la solution V, le pompage de la solution I-II et de la solution V dans le réacteur à écoulement continu, respectivement, le réglage d'une température de réaction, la régulation des débits de la solution I-II et de la solution V, la régulation du rapport de débits de la solution I-II à la solution V, et l'achèvement de la réaction dans le temps de séjour de réaction ; ledit réacteur à écoulement continu étant le micro-réacteur ; ledit rapport molaire de la *L*-cystéine telle que présentée dans la formule I au composé tel que présenté dans la formule II, la température de réaction, les débits de la solution I-II et de la solution V, le rapport de débits et le temps de séjour étant tous tels que décrits dans au moins l'une des revendications 1 à 8.

10. Procédé permettant la préparation selon au moins l'une des revendications 1 à 9, un post-traitement de la réaction de condensation comprenant les étapes suivantes : la dilution, l'ajustement de la valeur de pH à 2,5 à 3,0, le refroidissement, la recristallisation, la filtration et le séchage.

11. Procédé permettant la préparation selon la revendication 10, dans l'étape de dilution, un solvant utilisé pour diluer étant l'eau ; et/ou un rapport de masses volumiques du solvant de dilution à la *L*-cystéine telle que présentée dans la formule I valant de 7 à 10 ml/g ;
et/ou, dans l'étape d'ajustement de la valeur de pH à 2,5 à 3,0, un procédé d'ajustement de la valeur de pH à 2,5 à 3,0 consistant à utiliser un régulateur pour l'ajustement ; ledit régulateur étant de préférence un acide inorganique, en outre de préférence l'acide chlorhydrique ;
et/ou, avant l'étape d'ajustement de la valeur de pH à 2,5 à 3,0, une solution réactionnelle après dilution étant chauffée à 50 à 70°C ;
et/ou, dans l'étape de refroidissement, une température de refroidissement valant de 20 à 35°C ;
et/ou, dans l'étape de recristallisation, un solvant utilisé pour la recristallisation étant l'eau ; et/ou un rapport de masses volumiques du solvant utilisé pour la recristallisation au produit brut de la carbocystéine valant de 6 à 7 ml ; et/ou une température de recristallisation valant de 50 à 55°C ; et/ou la recristallisation comprenant en outre une étape de décoloration ; de préférence, après l'étape de décoloration, une valeur de pH de la solution recristallisée étant ajustée à 2 à 2,5.
